# EUROPEAN PATENT SPECIFICATION

(11) **EP 1 598 061 B1**
(45) Date of publication and mention of the grant of the patent: **18.08.2010**
(21) Application number: 05012137.5
(22) Date of filing: 12.06.1997
(51) Int. Cl.: A61K 9/20, A61K 47/10, A61K 47/26, A61J 3/10

(54) **Intraorally rapidly disintegrable tablet**
Eine im Mund schnell zerfallende Tablette
Comprimé intra-oral à désintégration rapide

(30) Priority: 14.06.1996 JP 15355396; 25.03.1997 JP 7110797
(43) Date of publication of application: 23.11.2005
(62) Divisional of application: 97927372.9
(73) Proprietor: Kyowa Hakko Kirin Co., Ltd., Tokyo 100-8185 (JP)
(72) Inventor: Ohta, Motohiro, Sunto-gun Shizuoka 411 (JP); Hayakawa, Eiji, Suntou-gun, Shizuoka 411-0942 (JP); Ito, Kunio, Sunto-gun Shizuoka 411 (JP); Tokuno, Sanji, Tokyo 142 (JP); Morimoto, Kiyoshi, Shizuoka 411-0808 (JP); Watanabe, Yasushi, Shizuoka 410 (JP)
(74) Representative: Vossius & Partner

(56) References cited:
- EP-A- 0 553 777
- GB-A- 2 224 207
- US-A- 3 885 026
- PATENT ABSTRACTS OF JAPAN vol. 012, no. 122 (C-488), 15 April 1988 (1988-04-15) & JP 62 242616 A (SANKYO CO LTD), 23 October 1987 (1987-10-23)
- PATENT ABSTRACTS OF JAPAN vol. 010, no. 339 (C-385), 15 November 1986 (1986-11-15) & JP 61 143316 A (CHUGAI PHARMACEUT CO LTD), 1 July 1986 (1986-07-01)

## Description

### Technical Field

This invention relates to a tablet rapidly disintegrable in an oral cavity.

### Background Art

There are various types of oral administrative medicines: tables, capsules, granules, powders, syrups and so on. However, such oral administrative medicines have several problems as follows. As to tablets and capsules, for example, it may be hard for aged persons or children whose swallowing power is weak to swallow them. And as to granules and powders, they may cause unpleasantness in a mouth after dosage or they may erroneously happen to enter into a respiratory tract or lungs. Further, they can not be taken where there is no water because water is usually required for dosage. As for syrups, it takes trouble for measuring on dosage and it is not be expected that aged persons or children measure them accurately. On the other hand, solid medicines which can be rapidly dissolved or disintegrated in an oral cavity can be taken without measuring or water, so they may be easily taken by such aged persons or children. Bv the way, there have been developed several types of medicines which can be rapidly dissolved or disintegrated in an oral cavity on dosing. For instance, in JP-B-82-50445, solid medicines which can be produced from water solution that mainly contains gelatin including an active ingredient by use of freeze drying method are disclosed. And in WO93/12789, solid medicines which can be produced by drying suspension including agar are also disclosed. However, the medicines produced by the above- mentioned prior method do not have enough hardness to be taken out by pushing from PTP packages (Frass Through Pack) containing the medicines. And, they require a special pharmaceutical manufacturing technology and also require an enormous investment in plants and equipments. JP-A-5-271054 and WO93/15724 disclose production methods of tables wherein tablets composed of saccaride are produced in such a manner that saccaride mixture supplied with appropriate water is compressed at a low pressure and then dried to make solid tablets. However, such methods also require a special pharmaceutical manufacturing technology and have the fear that powder composed of the tablets may adhere to the surface of a metal mold in compression process under moistening condition. It may be therefore difficult to utilize those methods for manufacturing on an industrial scale.

GB-A 2 224 207 discloses an oral pharmaceutical composition comprising piroxicam in micronised form of below 30 µm as the active ingredient together with lactose as a carrier.

TP-A-62-242616 pertains to a pharmaceutical composition containing loxoprofen sodium and additives so that the water absorption reaches 1.7 or more. The additives may be selected from, e.g., dextrin, hydroxypropylcellulose, hydroxypropylmethylcellulose, amicol, methylcellulose.

JP-A-61-143316 relates to the production of a tablet which is stable to molding pressure by tableting a mixture of the active ingredient and one or more sugars pulverized to a particle size ≤ 10 µm.

EP-A 0 553 777 discloses the manufacture of tablets comprising an active ingredient and a sugar alcohol having a preferred particle size of 30 µm to 50 µm. The optional inclusion of a disintegrant is also disclosed. The disintegrant encloses corn starch, potato starches, carmellose calcium, carmellose sodium and polyvinyl alcohol.

### Disclosure of the invention

The inventors of the present invention have examined an intraorally rapidly disintegrable tablet which does not require a special pharmaceutical manufacturing technology and can be simply and easily produced by a normal equipment. As a result, they have found that the compressed tablet consisting essentially of sugar alcohol or saccaride, such as D- mannitol or lactose, having an average particle diameter of not more than 30 µ m as a main ingredient, an active ingredient and a disintegrant can be disintegrated in a mouth within one minute and have hardness adequate for practical use, although such tablet has been considered unable to produce for a long time.

Accordingly, the present invention relates to a tablet comprising sugar alcohol or saccaride having an average particle diameter of not more than 30 µ m, an active ingredient and a disintegrant.

Further the present invention relates to a production method of a tablet characterized by compressing mixture which comprises sugar alcohol or saccaride each having an average particle diameter of not more than 30 µ m, an active ingredient, and a disintegrant.

In the present invention, such as D-mannitol.sorbitol, or the like, which is widely used for drugs and flood, can be used as sugar alcohol, and lactose and glucose or the like, which is also widely used for drugs and food, can be used as saccaride. At least one kind of sugar alcohol or saccharide is used.

As an active ingredient, followings are used, but other ingredients for oral administration can be also used.

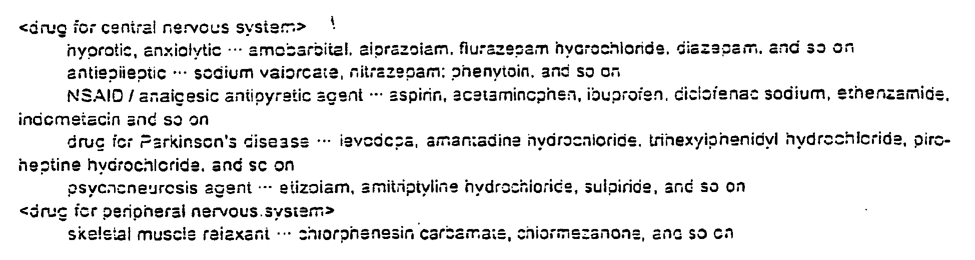

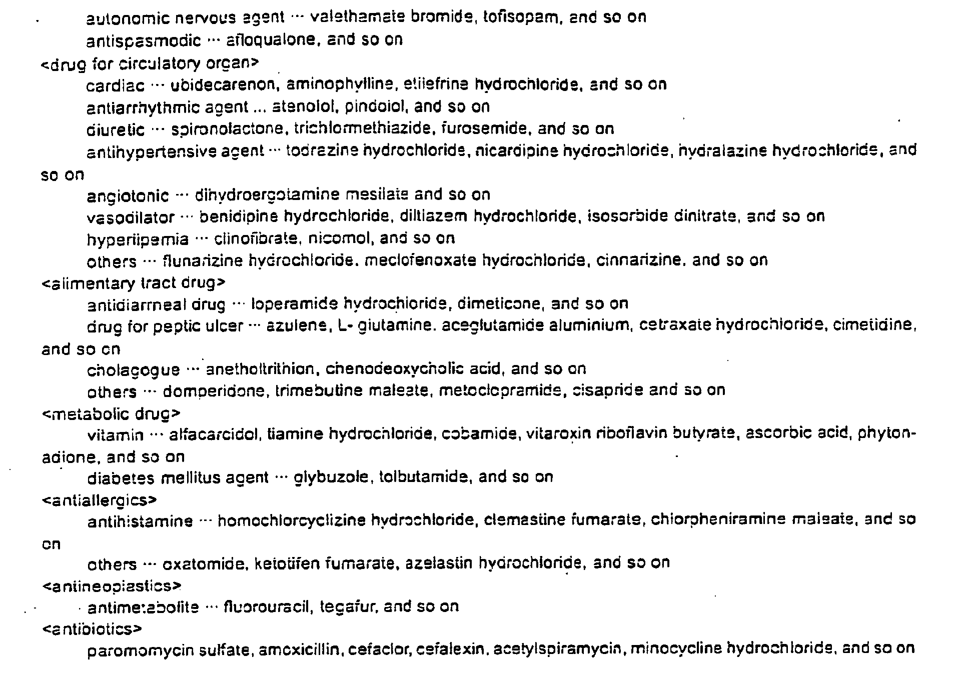

In the present invention, crosspovidone, crosscarmellose sodium, low substituted hydroxypropylcellulose or the like, which are widely used for drugs and food can be used as a disintegrant. At least one kind of disintegrant is used.

Next, a production method of a tablet according to the present invention will be described hereinafter

The tablet of the present invention can be obtained by compressing and tableting after granulating a mixture comprising sugar alcohol or saccaride having an average particle diameter of not more than 30 µm ground by means of a hammer mill or a jet mill or the like, an active ingredient, and a disintegrant. On the other hand, the tablet of the present invention also can be obtained by compressing and tableting after granulating a mixture comprising sugar alcohol or saccaride having an average particle diameter of not more than 30 µ m ground by means of a hammer mill, a jet mill or the like, an active ingredient, and a disintegrant under the presence of an easily volatile disintegrating adjuvant and thereafter the disintegrant adjuvant is volatilized.

The amount of sugar alcohol or saccaride is preferably about 60 - 95%, more preferably about 80 - 95% per one tablet.

The amount of active ingredient is different depending on the kind and dosage amount of active ingredients, however, 0.01 - 30% is preferable, and more preferably 0.01 - 10% per one tablet.

The amount of disintegrant present is preferably about 1 - 30mg per dosage, and more preferably 1 - 10% per one tablet.

Easily volatile disintegrating adjuvant is such as sublimative camphor, urethane, urea, ammonium bicarbonate, benzonic acid, or the like, however, camphor is most preferable. The amount of easily volatile disintegrating adjuvant is Preferably 1 - 20% and more preferably 1 - 10% per one tablet.

A wet granulation method using purified water, ethanol or the like can be preferably used. In the method, for example, granulation can be executed by means of a general granulator such as a fluid- bed granulator, a rotary stirring granulator or an extruding granulator. The granulated material is dried, and mixed with a lubricant, and thereafter compressed into predetermined shape. Binder, sour agent, foaming agent, sweetening agent, flavoring agent, or colorant can be added as additive. As a lubricant, such as magnesium stearate, stearic aid, stearyl alcohol, sucrose ester of fatty acid, talc, light anhydrous silicic acid, or like can be used. Binder is, for example, hydroxypropylcellulose, polyvinylpyrrolidone, hydroxypropylmethylcellulose, partially saponificated polyvinyl alcohol methylcellulose, pullulan or the like. Sour agent is citric acid, malic acid, adipic acid, ascorbic acid, or the like. Foaming agent is sodium bicarbonate, sodium carbonate, calcium carbonate, or the like. Sweetening agent is Aspartame (TM), saccharin, glycyrrhizic acid or the like. Flavoring agent is lemon, orange, pine, mint, menthol or the like. Colorant is yellow iron sesquioxide, red iron sesquioxide, tar color or the like. The amount of lubricant is preferably 0.01 - 1% and more preferably 0.01 - 0.5% per one tablet.

In particular the present invention provides the following:
1. A tablet comprising:
   sugar alcohol or saccharide each having an average particle diameter of not more than 30 µm,
   an active ingredient,
   a disintegrant, and
   a lubricant,
   wherein the tablet is obtainable by a process comprising (i) (ii) and (iii);
      (i) granulating a mixture comprising the sugar alcohol or the saccharide, the active ingredient and the disintegrant without any lubricant,
      (ii) drying the granulated mixture and
      (iii) compressing the granulated and dried mixture, the compressing process using a tabletting machine which is provided with punches and dies previously spread with the lubricant.
2. The tablet according to 1, wherein the amount of the sugar alcohol or the saccharide is 60 to 95 % by weight of the tablet.
3. The tablet according to 1 or 2, wherein the saccharide is lactose.
4. The tablet according to any of 1 to 3, wherein the disintegrant is selected from the group consisting of crospovidone, croscarmellose sodium, and low substituted hydroxypropylcellulose.
5. The tablet according to any of 1 to 4, wherein the granulation process is a wet granulation process in which purified water or ethanol is used.
6. The tablet according to any of 1 to 5, wherein the lubricant spread punches and dies is selected from the group consisting of magnesium stearate, stearic aid, stearyl alcohol, sucrose ester of fatty acid.
7. A process for producing a tablet, which comprises the steps of (i), (ii) and (iii):
   (i) granulating a mixture comprising sugar alcohol or saccharide, each having an average particle diameter of not more than 30 µm, an active ingredient and a disintegrant without any lubricant,
   (ii) drying the granulated mixture and
   (iii) compressing the granulated and dried mixture, the compressing step using a tabletting machine which is provided with punches and dies, previously spread with a lubricant.
8. The process according to 7, where the sugar alcohol or the saccharide is used in an amount of 60 to 95 % by weight of the tablet.
9. The process according to 7 or 8, wherein the saccharide is lactose.
10. The process according to any of 7 to 9, wherein the disintegrant is selected from the group consisting of crospovidone, croscarmellose sodium, and low substituted hydroxypropvicellulose.
11. The process according to any of 7 to 10, wherein the granulation process is a wet granulation process in which purified water or ethanol is used.
12. The process according to any of 7 to 11, wherein the lubricant spread punches and dies is selected from the group consisting of magnesium stearate, stearic aid, stearyl alcohol, sucrose ester of fatty acid.

Although a method of compression is not limited in the present invention, a rotary tablet machine, a hydraulic press machine or a single punch tableting machine which have high productivity can be more preferably used. When an easily volatile disintegrating adjuvant is used the tablet is dried by heating after compressing process. As to lubricants, they can be excluded from the granules in the granulation process, in that occasion it may be previously spread on the surfaces of punches and dies of a tableting machine before compression process. That makes the present invention more effective. Compression pressure of a rotary tablet machine may be preferably more than 300kg.

The shape of the tablet obtained in the present invention can be pills or other shapes such as a normal R surface tablet, a sugar coated R surface tablet, a tablet with square edges, a tablet with rounded edges, or a tablet with two R surfaces, or the like.

Further, the tablet may have a divisional line.

### Best Mode for Carrying Out the Invention

The present invention will be concretely explained according to examples and reference examples.

### Reference Example 1

1890g of D- mannitol (Towa Kasei Co., Ltd. : average particle diameter of about 60 µ m) and 100g of crosspovidone (POLYPLASOONE® XL-10 : GAF Co., Ltd.) were fed in a fluid- bed granulation dryer (Glatt Co., Ltd. : WSG-type 5), purified water was sprayed, then granulated material was obtained after granulation and drying processes. 10g of magnesium stearate was added and mixed with the granulated material, and they were compressed and tableted by a rotary tablet machine (Kikusui Seiko Co., Ltd., CLEAN PRESS COLLECT TYPE 12). Tableting conditions were as follows; tablet weight was 200mg, a metal mold was 8mm diameter flat- type, and compression pressure was varied such as 150kg, 300kg, 450kg, 600kg, and 800kg.

### Example 1 (Reference Example)

D- mannitol (Towa Kasei Co., Ltd. : average particle diameter of about 60 u m) was previously ground by a jet mill (Japan Pneumatic Co., Ltd.:type PJM-1-1.5) and the pulverized D- mannitol with average particle diameter of 20 µ m was obtained. 1890g of the pulverized D-mannitol and 100g of crosspovidone (POLYPLASDONE® XL-10 : GAF Co., Ltd.) was fed in a fluid- bed granulation dryer (Glatt Co., Ltd. : WSG- type 5), purified water was sprayed, and granulated material was obtained after granulation and drying processes. 10g of magnesium stearate was added and mixed with the granulated material, and they were compressed and tableted by a rotary tablet machine (Kikusui Seiko Co., Ltd, CLEAN PRESS COLLECT TYPE 12). Tableting conditions were the same as the referenc example 1.

### Reference Example 2

100g of domperidone, antiemetic agent, 1790g of lactose (DMV Co., Ltd. : average particle diameter of about 80 u m) and 100g of crosspovidone (POLYPLASDONc® XL-10 : GAF Co., Ltd.) were fed in a fluid- bed granulation dryer (Glatt Co., Ltd. : WSG-type 5), purified water was sprayed, and granulated material was obtained after granulation and drying processes. 10g of magnesium stearate was added and mixed with the granulated material, and they were compressed and tableted by a rotary tablet machine (Kikusui Seiko Co., Ltd., : CLEAN PRESS COLLECT type 12). Tableting conditions were the same as the reference example 1.

### Example 2 (Reference Example)

Lactose (DMV Co., Ltd.: average particle diameter of about 80 µ m) was previously ground by a jet mill (Japan Pneumatic Co., Ltd. : type PJM-1-1.5) and the pulverized lactose having average particle diameter of 15 µ m was obtained. 1790g of the pulverized lactose, 100g of domperidone, and 100g of crosspovidone (POLYPLASDONE® XL-10: GAF Co., Ltd.) were fed in a fluid- bed granulation dryer (Glatt Co., Ltd.: WSG-type 5), purified water was sprayed, and granulated material was obtained after granulation and drying processes. 10g of magnesium stearate was added and mixed with the granulated material, and they were compressed and tableted by a rotary tablet machine (Kikusui Seiko Co., Ltd., CLEAN PRESS COLLECT TYPE 12). Tableting conditions were the same as the reference example 1.

### Example 3

The granulated material obtained in the example 1 was tableted under the condition that tablet weight was 200mg, compression pressure was 50kg/c m², and a little magnesium stearate was coated on a metal mold (8mm diameter flat-type) and dies of a hydraulic press machine (Riken Seiki Co., Ltd.: type P-1B)

### Example 4 (Reference Example)

140g of the pulverized D- mannitol used in the example 1, 10g of domperidone, 10g of crosspovidone (POLYPLASDONE® XL-10 : GAF Co., Ltd.) and 40g of camphor were mixed in a vinyl bag and tableted under the condition that tablet weight was 200mg, a metal mold diameter was 9mm, a single punch tableting machine (Okada Seiko Co., Ltd.: N. 20E type both pressure powder tableting machine) was used, and compression pressure was 1500kg/cm². The compressed tablet was dried for 10 minutes at 80 °C under vacuum condition in a vacuum dryer.

Next, the hardness and disintegrating time of the tablet of the present invention will be described using an experimental example.

### Experimental Example

The hardness and the disintegrating time of the tablet obtained by the examples 1 and 2 and reference examples 1 and 2 were measured. The hardness of the tablet was measured by a tablet destructive strength measuring instrument (Toyama Sangyo Co., Ltd. : TH- 203CP type) The disintegrating time of the tablet was measured in such a way that the tablet was placed on a No. 10 wire cloth, water was dropped at a speed of 4ml/min. on the tablet, and the time till the tablet go through the wire cloth was measured. The time was determined as the disintegrating time.

The result is shown in a Table 1.

**Table 1**

| sample / compression pressure | | 150kg | 300kg | 450kg | 600kg | 800kg |
|---|---|---|---|---|---|---|
| comparison 1 | hardness | hard to be tableted | hard to be tableted | hard to be tableted | 1.9kgf | 2.3kgf |
| | disintegration | - | - | - | 20sec. | 22sec. |
| embodiment 1 | hardness | 1.9kgf | 3.9kgf | 5.1kgf | 6.2kgf | 7.3kgf |
| | disintegration | 10sec. | 15sec, | 16sec. | 19sec. | 27sec. |
| comparison 2 | hardness | hard to be tableted | hard to be tableted | hard to be tableted | 1,5kgf | 2.1kgf |
| | disintegration | - | - | - | 18sec. | 21sec. |
| embodiment 2 | hardness | 1.6kgf | 4.0kgf | 4.9kgf | 5.8kgf | 6.5kgf |
| | disintegration | 10sec. | 16sec. | 20sec. | 25sec. | 29sec. |

In reference examples 1 and 2, tableting was hard till 450kg compression pressure and tableting was made possible at around 600kg. However, the hardness of the tablet was not enough. In examples 1 and 2, enough tablet hardness could be obtained at more than 300kg compression pressure and the disintegrating time was very fast. When the tablet produced at 450kg compression pressure according to the example 1 was dosed, the tablet was disintegrated within 10 seconds in an oral cavity.

The tablets obtained by the examples 3 and 4 were also measured of its hardness and disintegrating time in the same way. The tablets obtained by the example 3 had enough hardness of about 6.5kgf and its disintegrating time was about 10 seconds. The hardness of the tablet of the example 4 was about 4kgf and its disintegrating time was about 2 seconds, which was very fast.

### Industrial Applicability

According to the present invention, a tablet rapidly disintegrable in an oral cavity can be provided.

## Claims

1. A tablet comprising:
sugar alcohol or saccharide each having an average particle diameter of not more than 30 µm,
an active ingredient,
a disintegrant, and
a lubricant,
wherein the tablet is obtainable by a process comprising (i), (ii) and (iii);
(i) granulating a mixture comprising the sugar alcohol or the saccharide, the active ingredient and the disintegrant without any lubricant,
(ii) drying the granulated mixture and
(iii) compressing the granulated and dried mixture, the compressing process using a tabletting machine which is provided with punches and dies previously spread with the lubricant.

2. The tablet according to claim 1, wherein the amount of the sugar alcohol or the saccharide is 60 to 95 % by weight of the tablet.

3. The tablet according to claim 1 or 2, wherein the saccharide is lactose.

4. The tablet according to any of claims 1 to 3, wherein the disintegrant is selected from the group consisting of crospovidone, croscarmellose sodium, and low substituted hydroxypropylcellulose.

5. The tablet according to any of claims 1 to 4, wherein the granulation process is a wet granulation process in which purified water or ethanol is used.

6. The tablet according to any of claims 1 to 5, wherein the lubricant spread on punches and dies is selected from the group consisting of magnesium stearate, stearic aid, stearyl alcohol and sucrose ester of fatty acid.

7. A process for producing a tablet, which comprises the steps of (i), (ii) and (iii):
(i) granulating a mixture comprising sugar alcohol or saccharide, each halving an average particle diameter of not more than 30 µm, an active ingredient and a disintegrant without any lubricant,
(ii) drying the granulated mixture and
(iii) compressing the granulated and dried mixture, the compressing step using a tabletting machine which is provided with punches and dies, previously spread with a lubricant.

8. The process according to claim 7, where the sugar alcohol or the saccharide is used in an amount of 60 to 95 % by weight of the tablet.

9. The process according to claim 7 or 8, wherein the saccharide is lactose.

10. The process according to any of claims 7 to, 9, wherein the disintegrant is selected from the group consisting of crospovidone, croscarmellose sodium, and low substituted hydroxypropylcellulose.

11. The process according to any of claims 7 to 10, wherein the granulation process is a wet granulation process in which purified water or ethanol is used.

12. The process according to any of claims 7 to 11, wherein the lubricant spread on punches and dies is selected from the group consisting of magnesium stearate, stearic aid, stearyl alcohol and sucrose ester of fatty acid.

## Patentansprüche

1. Tablette, die folgendes umfasst:
Zuckeralkohol oder Saccharid mit jeweils einem mittleren Partikeldurchmesser von nicht mehr als 30 µm,
einen Wirkstoff,
ein Sprengmittel und
ein Gleitmittel,
wobei die Tablette durch ein Verfahren erhältlich ist, das (i), (ii) und (iii) umfasst;
(i) Granulieren einer Mischung, die den Zuckeralkohol oder das Saccharid, den Wirkstoff und das Sprengmittel ohne jegliches Gleitmittel umfasst,
(ii) Trocknen der granulierten Mischung und
(iii) Komprimieren der granulierten und getrockneten Mischung, wobei im Kompressionsverfahren eine Tablettiermaschine verwendet wird, die mit Stempeln und Matrizen ausgestattet ist, auf denen vorher das Gleitmittel verteilt wurde.

2. Tablette nach Anspruch 1, wobei der Zuckeralkohol oder das Saccharid in einer Menge von 60 bis 95 Gew.-% der Tablette vorliegt.

3. Tablette nach Anspruch 1 oder 2, wobei das Saccharid Lactose ist.

4. Tablette nach einem der Ansprüche 1 bis 3, wobei das Sprengmittel aus der Gruppe ausgewählt ist, die aus Crospovidon, Croscarmellose-Natrium und niedrig substituierter Hydroxypropylcellulose besteht.

5. Tablette nach einem der Ansprüche 1 bis 4, wobei das Granulierverfahren ein Nassgranulierverfahren ist, in dem gereinigtes Wasser oder Ethanol verwendet wird.

6. Tablette nach einem der Ansprüche 1 bis 5, wobei das auf Stempeln und Matrizen verteilte Gleitmittel ausgewählt ist aus der Gruppe bestehend aus Magnesiumstearat, Stearinsäure, Stearylalkohol und Fettsäuresaccharoseester.

7. Verfahren zum Herstellen einer Tablette, das die Schritte (i), (ii) und (iii) umfasst:
(i) Granulieren einer Mischung, die einen Zuckeralkohol oder ein Saccharid, die jeweils einen mittleren Partikeldurchmesser von nicht mehr als 30 µm aufweisen, einen Wirkstoff und ein Sprengmittel ohne jegliches Gleitmittel umfasst,
(ii) Trocknen der granulierten Mischung und
(iii) Komprimieren der granulierten und getrockneten Mischung, wobei im Kompressionsschritt eine Tablettiermaschine verwendet wird, die mit Stempeln und Matrizen ausgestattet ist, auf denen vorher ein Gleitmittel verteilt wurde.

8. Verfahren nach Anspruch 7, wobei der Zuckeralkohol oder das Saccharid in einer Menge von 60 bis 95 Gew.-% der Tablette verwendet wird.

9. Verfahren nach Anspruch 7 oder 8, wobei das Saccharid Lactose ist.

10. Verfahren nach einem der Ansprüche 7 bis 9, wobei das Sprengmittel aus der Gruppe ausgewählt ist, die aus Crospovidon, Croscarmellose-Natrium und niedrig substituierter Hydroxypropylcellulose besteht.

11. Verfahren nach einem der Ansprüche 7 bis 10, wobei das Granulierverfahren ein Nassgranulierverfahren ist, in dem gereinigtes Wasser oder Ethanol verwendet wird.

12. Verfahren nach einem der Ansprüche 7 bis 11, wobei das auf Stempeln und Matrizen verteilte Gleitmittel ausgewählt ist aus der Gruppe bestehend aus Magnesiumstearat, Stearinsäure, Stearylalkohol und Fettsäuresaccharoseester.

## Revendications

1. Comprimé comprenant :
un alcool de sucre ou un saccharide ayant chacun un diamètre de particule moyen de pas plus de 30 µm,
un principe actif,
un désintégrant, et
un lubrifiant,
dans lequel le comprimé est susceptible d'être obtenu au moyen d'un procédé comprenant (i), (ii) et (iii) :
(i) granulation d'un mélange comprenant l'alcool de sucre ou le saccharide, le principe actif et le désintégrant sans lubrifiant,
(ii) séchage du mélange granulé et
(iii)compression du mélange granulé et séché, le procédé de compression utilisant une machine à comprimer qui est fournie avec des poinçons et des matrices préalablement recouvert de lubrifiant.

2. Comprimé selon la revendication 1, dans lequel la quantité d'alcool de sucre ou de saccharide est de 60 à 95% en poids du comprimé.

3. Comprimé selon la revendication 1 ou 2, dans lequel le saccharide est le lactose.

4. Comprimé selon l'une quelconque des revendications 1 à 3, dans lequel le désintégrant est choisi dans le groupe constitué de crospovidone, croscarmellose sodique, et d'hydroxypropylcellulose faiblement substitué.

5. Comprimé selon l'une quelconque des revendications 1 à 4, dans lequel le procédé de granulation est un procédé de granulation humide dans lequel de l'eau ou de l'éthanol purifié est utilisé.

6. Comprimé selon l'une quelconque des revendications 1 à 5, dans lequel le lubrifiant qui recouvre les poinçons et les matrices est choisi dans le groupe constitué de stéarate de magnésium, d'acide stéarique, d'alcool stéarylique, d'ester de sucrose d'acide gras.

7. Procédé de production d'un comprimé, qui comprend les étapes (i), (ii) et (iii) :
(i) granulation d'un mélange comprenant l'alcool de sucre ou le saccharide, ayant chacun un diamètre de particule moyen de pas plus de 30 µm, un principe actif et un désintégrant sans lubrifiant,
(ii) séchage du mélange granulé et
(iii)compression du mélange granulé et séché, le procédé de compression utilisant une machine à comprimer qui est fournie avec des poinçons et des matrices préalablement recouvert de lubrifiant.

8. Procédé selon la revendication 7, où l'alcool de sucre ou le saccharide est utilisé en une quantité de 60 à 95% en poids du comprimé.

9. Procédé selon la revendication 7 ou 8, dans lequel le saccharide est le lactose.

10. Procédé selon l'une quelconque des revendications 7 à 9, dans lequel le désintégrant est choisi dans le groupe constitué de crospovidone, croscarmellose sodique, et d'hydroxypropylcellulose faiblement substitué.

11. Procédé selon l'une quelconque des revendications 7 à 10, dans lequel le procédé de granulation est un procédé de granulation humide dans lequel de l'eau ou de l'éthanol purifié est utilisé.

12. Procédé selon l'une quelconque des revendications 7 à 11, dans lequel le lubrifiant qui recouvre les poinçons et les matrices est choisi dans le groupe constitué de stéarate de magnésium, d'acide stéarique, d'alcool stéarylique, d'ester de sucrose d'acide gras.
